# EUROPÄISCHE PATENTSCHRIFT

(11) **EP 0 771 818 B1**
(45) Veröffentlichungstag und Bekanntmachung des Hinweises auf die Patenterteilung: **03.04.2002**
(21) Anmeldenummer: 96114454.0
(22) Anmeldetag: 10.09.1996
(51) Int. Cl.: C07K 5/10, C07K 14/75, C07K 14/78, A61K 38/04, G01N 33/68

(54) **Biotinderivate**
Biotin derivatives
Dérivés de biotine

(30) Priorität: 14.09.1995 DE 19534016
(43) Veröffentlichungstag der Anmeldung: 07.05.1997
(73) Patentinhaber: MERCK PATENT GmbH, 64293 Darmstadt (DE)
(72) Erfinder: Jonczyk, Alfred, Dr., 64295 Darmstadt (DE); Goodman, Simon, Dr., 64287 Darmstadt (DE); Diefenbach, Beate, Dr., 64289 Darmstadt (DE); Kessler, Horst prof., 85748 Garching (DE); Finsinger, Dirk, 85748 Garching (DE)

(56) Entgegenhaltungen:
- KIDNEY INTERNATIONAL, Bd. 46 , Nr. 4, Oktober 1994, NEW YORK, Seiten 1050-1058, XP000670848 E NOIRI ET AL.: "Cyclic RGD peptides ameliorate ischemic acute renal failure in rats"
- BIOCHEMICAL AND BIOPHYSICAL RESEARCH COMMUNICATIONS, Bd. 181, Nr. 3, 31.Dezember 1991, ORLANDO, FL US, Seiten 1245-1250, XP002029500 H I MAGAZINE ET AL.: "Evaluation of endothelin receptor populations using endothelin-1 biotinylated at lysine-9 side chain "

## Beschreibung

Die Erfindung betrifft Verbindungen ausgewählt aus der Gruppe
Bit-Gly-Gly-Gly-Arg-Gly-Asp-Ser-Pro-Lys-OH,
Bit-Gly-Gly-Gly-Lys-Thr-Ala-Asp-Cys(Trt)-Pro-OH,
cyclo-(Arg-Gly-Asp-D-Phe-Lys(N^{ε}-Bit)),
cyclo-(Arg-Gly-Asp-D-Phe-Lys(N^{ε}-Aha)),
cyclo-(Arg-Gly-Asp-D-Phe-Lys(N^{ε}-Bit-Aha)-Gly),
cyclo-(Arg-Gly-Asp-D-Phe-Val-Lys(N^{ε}-Bit-Aha)),
cyclo-(Arg-Gly-Asp-D-Phe-N-Me-Lys(N^{ε}-Bit-Aha)),
cyclo-(Arg-Gly-Asp-D-Phe-Lys(N^{ε}-Bit-Aha)),
cyclo-(Arg-Gly-Asp-D-Phe-Lys(BOC-Aha)),
sowie deren Salze.

E. Nori et al. beschreibt in Kidney International, Bd. 46, Nr. 4, Oktober 1994, 1050-1058, cyclische RGD-Peptide, u.a. GRGDSPK-Biot, als Inhibitoren der Zell-Zell-Adhäsion.
Ähnliche Verbindungen biotinylierter Peptide sind z. B. beschrieben in WO 9415956 (biotinylierte Endothelin Rezeptor Antagonisten), in WO 9413313 (biotinylierte LHRH Antagonisten) oder in WO 9418325 (biotinylierter Nekrosefaktor).
Die Biotinylierung von Peptiden während der Festphasensynthese am Harz, zum Zwecke der verbesserten Reinigungsmöglichkeit, ist von T.J. Lobl et al. in Anal. Biochem. 170 , 502 (1988) beschrieben. Ähnliche Verbindungen cyclischer und linearer Peptide sind aus DE 43 10 643, DE 43 36 758, EP 0 406 428 und WO 89/05150 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die erfindungsgemäßen Verbindungen und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der αᵥ-, β₃- oder β₅-Integrin-Rezeptoren mit Liganden hemmen, wie z. B. die Bindung von Fibrinogen an den β₃-Integrinrezeptor. Besondere Wirksamkeit zeigen die Verbindungen im Fall der Integrine αᵥβ₃, αᵥβ₅, α_{IIb}β₃ sowie αᵥβ₁, αᵥβ₆ und αᵥβ₈.
Diese Wirkung kann z.B. nach der Methode nachgewiesen werden, die von J.W. Smith et al. in J. Biol. Chem. 265, 12267-12271 (1990) beschrieben wird.
Die Abhängigkeit der Entstehung von Angiogenese von der Wechselwirkung zwischen vaskulären Integrinen und extrazellulären Matrix-proteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptosis (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Die erfindungsgemäßen Verbindungen, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:
Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Die erfindungsgemäßen Verbindungen können als Arzneimittelwirkstoffe in der Human- und Veterinärmedizin eingesetzt werden, insbesondere zur Prophylaxe und/oder Therapie von Thrombose, myocardialem Infarkt, Arteriosklerose, Entzündungen, Apoplexie, Angina pectoris, Tumorerkrankungen, osteolytischen Krankheiten wie Osteoporose, pathologisch angiogenen Krankheiten wie z. B. Entzündungen, ophthalmologischen Krankheiten, diabetischer Retinopathie, makularer Degeneration, Myopia, okularer Histoplasmose, rheumatischer Arthritis, Osteoarthritis, rubeotischem Glaukom, ulcerativer Colitis, Morbus Crohn, Atherosklerose, Psoriasis, Restenose nach Angioplastie, viraler Infektion, bakterieller Infektion, Pilzinfektion, bei akutem Nierenversagen und bei der Wundheilung zur Unterstützung der Heilungsprozesse.

Die erfindungsgemäßen Verbindungen können als antimikrobiell wirkende Substanzen bei Operationen eingesetzt werden, wo Biomaterialien, Implantate, Katheter oder Herzschrittmacher eingesetzt werden.
Dabei wirken sie antiseptisch. Die Wirksamkeit der antimikrobiellen Aktivität kann durch das von P.Valentin-Weigund et al., in Infection and Immunity, 2851-2855 (1988) beschriebene Verfahren nachgewiesen werden.

Da die erfindungsgemäßen Verbindungen Inhibitoren der Fibrinogenbindung und damit Liganden der Fibrinogenrezeptoren auf Blutplättchen darstellen, können sie als Diagnostika zur Detektion und Lokalisierung von Thromben im vaskulären System *in vivo* verwendet werden, da der Biotinylrest einen UV-detektierbaren Rest darstellt.

Die erfindungsgemäßen Verbindungen können als Inhibitoren der Fibrinogenbindung auch als wirksame Hilfsmittel zum Studium des Metabolismus von Blutplättchen in unterschiedlichen Aktivierungsstadien oder von intrazellulären Signalmechanismen des Fibrinogenrezeptors verwendet werden. Die detektierbare Einheit des "Biotinlabels" erlaubt es, nach Bindung an den Rezeptor, genannte Mechanismen zu untersuchen.

Die vor- und nachstehend aufgeführten Abkürzungen von Aminosäureresten stehen für die Reste folgender Aminosäuren:
- Abu: 4-Aminobuttersäure
- Aha: 6-Aminohexansäure, 6-Aminocapronsäure
- Ala: Alanin
- Asn: Asparagin
- Asp: Asparaginsäure
- Arg: Arginin
- Cys: Cystein
- Dab: 2,4-Diaminobuttersäure
- Dap: 2,3-Diaminopropionsäure
- Gln: Glutamin
- Glp: Pyroglutaminsäure
- Glu: Glutaminsäure
- Gly: Glycin
- His: Histidin
- homo-Phe: homo-Phenylalanin
- Ile: Isoleucin
- Leu: Leucin
- Lys: Lysin
- Met: Methionin
- Nle: Norleucin
- Orn: Ornithin
- Phe: Phenylalanin
- Phg: Phenylglycin
- 4-Hal-Phe: 4-Halogen-phenylalanin
- Pro: Prolin
- Ser: Serin
- Thr: Threonin
- Trp: Tryptophan
- Tyr: Tyrosin
- Val: Valin
sowie

### Bit

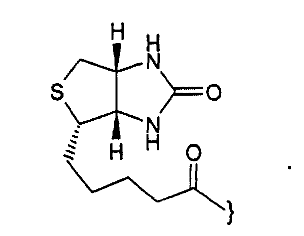

Ferner bedeuten nachstehend:
- BOC: tert.-Butoxycarbonyl
- CBZ: Benzyloxycarbonyl
- DCCI: Dicyclohexylcarbodiimid
- DMF: Dimethylformamid
- Et: Ethyl
- Fmoc: 9-Fluorenylmethoxycarbonyl
- HOBt: 1 -Hydroxybenzotriazol
- Me: Methyl
- MBHA: 4-Methyl-benzhydrylamin
- Mtr: 4-Methoxy-2,3,6-trimethylphenyl-sulfonyl
- OBut: tert.-Butylester
- OMe: Methylester
- OEt: Ethylester
- POA: Phenoxyacetyl
- TFA: Trifluoressigsäure
- Trt: Trityl (Triphenylmethyl).

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die erfindungsgemäßen Verbindungen können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der erfindungsgemäßen Verbindungen aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, femer auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCI in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Die Tritylgruppe wird zum Schutz der Aminosäuren Histidin, Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammoniumformiat (anstelle von Wasserstoff) an Pd/C in Methanol/DMF bei 20-30°.

Eine erfindungsgemäße Base kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, femer organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine erfindungsgemäße Säure durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyl- oder Diisopropyl-ammoniumsalze, Monoethanol-, Diethanol- oder Diisopropylammoniumsalze, Cyclohexyl-, Dicyclohexylammoniumsalze, Dibenzylethylendiammoniumsalze, weiterhin z. B. Salze mit Arginin oder Lysin.

Gegenstand der Erfindung sind ferner pharmazeutische Zubereitungen, enthaltend mindestens eine erfindungsgemäße Verbindung und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine. Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Die erfindungsgemäßen Verbindungen und ihre physiologisch unbedenklichen Salze können als Integrininhibitoren bei der Bekämpfung von Krankheiten, insbesondere von pathologisch angiogenen Erkrankungen, Thrombosen, Herzinfarkt, koronaren Herzerkrankungen, Arteriosklerose, Tumoren, Osteoporose, Entzündungen und Infektionen verwendet werden.

Dabei können die erfindungsgemäßen Substanzen in der Regel in Analogie zu anderen bekannten, im Handel befindlichen Peptiden, insbesondere aber in Analogie zu den in der US-A-4 472 305 beschriebenen Verbindungen verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die neuen Verbindungen in der analytischen Biologie und Molekularbiologie verwendet werden. Hierbei wird die Fähigkeit der Komplexbildung zwischen dem Biotinylrest und dem Glycoprotein Avidin ausgenutzt.
Bekannt sind Verwendungen des Biotin-Avidin-Komplexes aus E.A.Bayer und M.Wilchek in Methods of Biochemical Analysis 26, 1-45 (1980) (Lit.1).

Die neuen Verbindungen können als Integrinliganden zur Herstellung von Säulen für die Affinitätschromatographie zur Reindarstellung von Integrinen verwendet werden. Der Komplex aus einem Avidinderivatisierten Trägermaterial, z.B. Sepharose und den neuen Verbindungen wird nach an sich bekannten Methoden gebildet, wie dies z. B. in Lit.1 beschrieben ist.
Aus diesem Grunde wird an dieser Stelle nicht weiter auf diese Methode eingegangen und auf die entsprechende Literatur, z. B. Lit.1 verwiesen.

Als polymere Trägermaterialien eignen sich die an sich in der Peptidchemie bekannten polymeren festen Phasen mit vorzugsweise hydrophilen Eigenschaften, beispielsweise quervernetzte Polyzucker wie Cellulose, Sepharose oder Sephadex^{R}, Acrylamide, Polymer auf Polyethylenglykolbasis oder Tentakelpolymere^{R}.

Die neuen Verbindungen können auch als diagnostische Marker für Anti-Biotin-Antikörper-Reaktionen in ELISA-typ Assays und in der FACS (**F**luorescence **A**ctivated **C**ell **S**orter)-Analyse verwendet werden.
Bekannt ist die Verwendung von Antibiotin-Antikörpern zur Detektion von Biotin durch M. Berger, Biochemistry 14, 2338-2342 (1975).
Der Einsatz von mit Biotin derivatisiertem Immunglobulin IgG in einem Enzymimmunoassay (ELISA) ist von U. Holmkov-Nielsen et al. in Journal of Chromatography, 297, 225-233 (1984) beschrieben.
Im J. Immunol. Methods 181, 55-64 (1995) ist von J. Gao und S.J. Shattil ein ELISA-Test beschrieben, der Substanzen nachweist, die die Integrin α_{IIb}β_{III} Aktivierung inhibieren. Zum Nachweis wird hier biotinyliertes Fibrinogen eingesetzt.

Die Anwendung der Durchflußzytometrie in der Klinischen Zelldiagnostik ist beschrieben von G. Schmitz und G. Rothe in DG Klinische Chemie Mitteilungen 24 (1993) Heft 1, Seite 1-14.

Weiterhin können die erfindungsgemäßen Verbindungen in der Kraftfeldmikroskopie (atomic force microscopy AFM) zur Messung der Stärke von Ligand-Rezeptor-Wechselwirkungen eingesetzt werden.
Ligand bedeutet vorzugsweise einen Komplex aus Avidin und den neuen Verbindungen.
Rezeptor bedeutet vorzugsweise einen Integrinrezeptor.
Von E.-L. Florin et al. sind Messungen von Adhäsionskräften zwischen einem Avidin-funktionalisierten Kraftfeldmikroskop und biotinylierter Agarose in Science 264, 415-417 (1994) beschrieben.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

RZ = Retentionszeit (Minuten) bei HPLC in den folgenden Systemen:

### [A]

| | |
|---|---|
| Säule | Nucleosil 7C18 250 x 4 mm |
| Eluent A | 0,1 % TFA in Wasser |
| Eluent B | 0,1 % TFA in Acetonitril |
| Fluß | 1 ml/min |
| Gradient | 20 - 50 % B / 30 min. |

### [B]

50 minütiger Gradient von 0-80 % 2-Propanol in Wasser mit 0,3 % TFA bei 1 ml/min auf einer Säule Lichrosorb^{R} RP Select B (7 µm) 250 x 4 mm

### [C]

| | |
|---|---|
| Säule | Lichrospher (5 µm) 100RP8 125 x 4 mm |
| Eluent A | 0,01 M Na-Phosphat pH 7.0 |
| Eluent B | 0,005 M Na-Phosphat pH 7.0 / 60 Vol. % 2-Propanol |
| Fluß | 0,7 ml/min |
| Gradient | 1 - 99 % B / 50 min. |

- Massenspektrometrie (MS):: El (Elektronenstoß-lonisation) M⁺
FAB (Fast Atom Bombardment) (M+H)⁺

DMPP-Harz steht für 4-(2',4'-Dimethoxyphenyl-hydroxymethyl)phenoxy-Harz, ein supersäurelabiles Harz, welches die Synthese von seitenkettengeschützten Peptiden erlaubt.

### Beispiel 1

0,6 g Fmoc-Lys(Boc)-OH werden in 100 ml Dichlormethan gelöst, 1,2 Äquivalenten DMPP-Harz, 1,4 Äquivalenten HOBt und 1,4 Äquivalenten DCCI versetzt und 12 Stunden bei Raumtemperatur gerührt. Nach Entfernung des Lösungsmittels erhält man Fmoc-Lys(Boc)-DMPP-Harz. Im Peptid-Synthesizer kondensiert man Fmoc-Pro-OH mit H-Lys(BOC)-DMPP-Harz [Freisetzung aus Fmoc-Lys(BOC)-DMPP-Harz mit Piperidin/DMF (20 %ig)], indem man den dreifachen Überschuß des geschützten Prolins einsetzt. Die Kupplung wird mit DCCI/HOBt bei Raumtemperatur durchgeführt. Man erhält Fmoc-Pro-Lys(BOC)-DMPP-Harz. Analog erhält man durch anschließende Abspaltung der Fmoc-Schutzgruppe und aufeinanderfolgende Kupplungen mit Fmoc-Ser(But)-OH, Fmoc-Asp(OBut)-OH, Fmoc-Gly-OH, Fmoc-Arg(Mtr)-OH, Fmoc-Gly-OH, Fmoc-Gly-OH, Fmoc-Gly-OH und Bit-OH unter sich bei jeder Kupplung wiederholenden Reaktionsbedingungen
- Freisetzung der α-Aminogruppe mit Piperidin/DMF (20 %ig)
- Waschen mit Dimethylacetamid
- Umsetzung mit der Fmoc-Aminosäure bzw. Bit-OH

Bit-Gly-Gly-Gly-Arg(Mtr)-Gly-Asp(OBut)-Ser(But)-Pro-Lys(BOC)-DMPP-Harz.
Man wäscht das Harz mit CF₃SO₃H/CH₂Cl₂/H₂O und erhält Bit-Gly-Gly-Gly-Arg(Mtr)-Gly-Asp(OBut)-Ser(But)-Pro-Lys(BOC)-OH.
Nach Abspaltung der Schutzgruppen mit 2 N HCI in Dioxan, Entfernen des Lösungsmittels, Aufnahme des Rückstands in TFA/CH₂Cl₂ und Ausfällen mit Et₂O reinigt man über RP-HPLC. Man erhält
Bit-Gly-Gly-Gly-Arg-Gly-Asp-Ser-Pro-Lys-OH x 2 TFA; RZ [B] = 12.14; FAB 1056.

### Beispiel 2

Analog Beispiel 1 erhält man durch aufeinanderfolgende Kupplungen des DMPP-Harzes mit Fmoc-Pro-OH, Fmoc-Cys(Trt)-OH, Fmoc-Asp(OBut)-OH, Fmoc-Ala-OH, Fmoc-Thr(But)-OH, Fmoc-Lys(BOC)-OH, Fmoc-Gly-OH, Fmoc-Gly-OH, Fmoc-Gly-OH und Bit-OH:

Bit-Gly-Gly-Gly-Lys(BOC)-Thr(But)-Ala-Asp(OBut)-Cys(Trt)-Pro-DMPP-Harz.
Durch Abspaltung vom Harz, Abspaltung der Schutzgruppen und Aufreinigung erhält man
Bit-Gly-Gly-Gly-Lys-Thr-Ala-Asp-Cys(Trt)-Pro-OH x 2 TFA; RZ [B] 27.6; FAB 1273.

### Beispiel 3

Zu einer Lösung aus 3,05 g Cyclo-(Arg-Gly-Asp-D-Phe-Lys) [erhältlich durch Cyclisierung von H-Arg(Mtr)-Gly-Asp(OBut)-D-Phe-Lys(BOC)-OH zu Cyclo-(Arg(Mtr)-Gly-Asp(OBut)-D-Phe-Lys(BOC)) und anschließender Abspaltung der Schutzgruppen] in 100 ml Dichlormethan gibt man 1,7 g wohlfeil erhältlichen (+)-Biotinyl-N-succinimidylester und 0,5 g Triethylamin.
Man rührt 5 Stunden bei Raumtemperatur und erhält nach üblicher Aufarbeitung Cyclo-(Arg-Gly-Asp-D-Phe-Lys(N^{ε}-Bit)) x TFA; RZ [B] 11.32; FAB 830.

### Beispiel 4

Analog Beispiel 3 erhält man aus 3,05 g Cyclo-(Arg-Gly-Asp-D-Phe-Lys) und 2,3 g wohlfeilem (+)-Biotinyl-6-aminocapronsäure-N-succinimidylester ("A") und 0,5 g Triethylamin Cyclo-(Arg-Gly-Asp-D-Phe-Lys(N^{ε}-Bit-Aha)) x TFA; RZ [C] 23.67; FAB 943.

### Beispiel 5

Zu einer Lösung von 3,05 g Cyclo-(Arg-Gly-Asp-D-Phe-Lys) in 40 ml 5 %iger wässriger NaHCO₃ und 40 ml THF gibt man 6g Boc-Aha-N-succinimidylester. Man rührt 4 Stunden, arbeitet wie üblich auf und erhält Cyclo-(Arg-Gly-Asp-D-Phe-Lys(BOC-Aha)); RZ [C] 27.7; FAB 817. Nach Abspaltung der BOC-Gruppe in HCl/Dioxan erhält man nach üblicher Aufarbeitung Cyclo-(Arg-Gly-Asp-D-Phe-Lys(N^{ε}-Aha)) x 2 TFA; RZ [C] 14.76; FAB 717.
Analog zu Beispiel 1 erhält man durch anschließende Umsetzung mit (+)-Biotinyl-N-succinimidylester Cyclo-(Arg-Gly-Asp-D-Phe-Lys(N^{ε}-Bit-Aha)) x TFA; RZ [C] 23.67; FAB 943.

### Beispiel 6

Analog Beispiel 4 erhält man aus Cyclo-(Arg-Gly-Asp-D-Phe-Lys-Gly) [erhältlich durch Cyclisierung von H-Arg(Mtr)-Gly-Asp(OBut)-D-Phe-Lys(BOC)-Gly-OH zu
Cyclo-(Arg(Mtr)-Gly-Asp(OBut)-D-Phe-Lys(BOC)-Gly) und anschließender Abspaltung der Schutzgruppen] und (+)-Biotinyl-6-aminocapronsäure-N-succinimidylester
Cyclo-(Arg-Gly-Asp-D-Phe-Lys(N^{ε}-Bit-Aha)-Gly) x TFA; RZ [A] 10.97; FAB 1000.

Analog erhält man aus Cyclo-(Arg-Gly-Asp-D-Phe-Val-Lys) [erhältlich durch Cyclisierung von
H-Arg(Mtr)-Gly-Asp(OBut)-D-Phe-Val-Lys(BOC)-OH zu Cyclo-(Arg(Mtr)-Gly-Asp(OBut)-D-Phe-Val-Lys(BOC)) und anschließender Abspaltung der Schutzgruppen] und (+)-Biotinyl-6-aminocapronsäure-N-succinimidylester
Cyclo-(Arg-Gly-Asp-D-Phe-Val-Lys(N^{ε}-Bit-Aha)) x TFA; RZ [A] 16.11; FAB 1042.

Analog erhält man aus Cyclo-(Arg-Gly-Asp-D-Phe-N-Me-Lys) und (+)-Biotinyl-6-aminocapronsäure-N-succinimidylester Cyclo-(Arg-Gly-Asp-D-Phe-N-Me-Lys(N^{ε}-Bit-Aha)).

### Beispiel 7

Herstellung eines für die Affinitätschromatographie zur Integrinreinigung geeigneten Materials:

Die Aktivierung von Sepharose erfolgt wie in Lit. 1, Seite 14 beschrieben. Danach werden 20 mg Avidin in 20 ml 0,1 M Natriumbicarbonatlösung zu 10 g aktivierter Sepharose gegeben.
Die Suspension wird 12 Stunden bei 4° gerührt und danach gewaschen. Das Material wird danach in Wasser mit einigen Kristallen Natriumazid suspendiert.
Die Bildung des Avidin-Komplexes mit den biotinylierten erfindungsgemäßen Verbindungen z. B. Cyclo-(Arg-Gly-Asp-D-Phe-Lys(N^{ε}-Bit)) x TFA erfolgt, indem man 1,1 Äquivalent Peptid in Natriumacetatpuffer löst, die Lösung zu der Suspension von Avidin-Sepharose gibt und 10 Stunden bei 4° rührt. Überschüssiges Peptid wird durch Waschen entfernt.

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg eines erfindungsgemäßen Wirkstoffes, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg eines erfindungsgemäßen Wirkstoffes werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g eines erfindungsgemäßen Wirkstoffes in 10 l isotonischer NaCI-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen ausgewählt aus der Gruppe
Bit-Gly-Gly-Gly-Arg-Gly-Asp-Ser-Pro-Lys-OH,
Bit-Gly-Gly-Gly-Lys-Thr-Ala-Asp-Cys(Trt)-Pro-OH,
cyclo-(Arg-Gly-Asp-D-Phe-Lys(N^{ε}-Bit)),
cyclo-(Arg-Gly-Asp-D-Phe-Lys(N^{ε}-Aha)),
cyclo-(Arg-Gly-Asp-D-Phe-Lys(N^{ε}-Bit-Aha)-Gly),
cyclo-(Arg-Gly-Asp-D-Phe-Val-Lys(N^{ε}-Bit-Aha)),
cyclo-(Arg-Gly-Asp-D-Phe-N-Me-Lys(N^{ε}-Bit-Aha)),
cyclo-(Arg-Gly-Asp-D-Phe-Lys(N^{ε}-Bit-Aha)),
cyclo-(Arg-Gly-Asp-D-Phe-Lys(BOC-Aha)),
sowie deren Salze.

2. Pharmazeutische Zubereitung, **gekennzeichnet durch** einen Gehalt an mindestens einer Verbindung nach Anspruch 1 und/oder einem ihrer physiologisch unbedenklichen Salze.

3. Verbindungen nach Anspruch 1 und ihre physiologisch
unbedenklichen Salze als Integrininhibitoren zur Bekämpfung von pathologisch angiogenen Erkrankungen, Thrombosen, Herzinfarkt, koronaren Herzerkrankungen, Arteriosklerose, Tumoren, Osteoporose, Entzündungen und Infektionen.

4. Verwendung von Verbindungen nach Anspruch 1 und/oder ihre physiologisch unbedenklichen Salze zur Herstellung eines Arzneimittels.

5. Verwendung von Verbindungen nach Anspruch 1 zur Reinigung von Integrinen durch Affinitätschromatographie.

6. Verwendung von Verbindungen nach Anspruch 1 als diagnostische Marker für Anti-Biotin-Antikörper-Reaktionen in ELISA-typ Assays und in der FACS-Analyse.

7. Verwendung von Verbindungen nach Anspruch 1 in der Kraftfeldmikroskopie zur Messung der Stärke von Ligand-Rezeptor-Wechselwirkungen.

## Claims

1. Compounds selected from the group consisting of
Bit-Gly-Gly-Gly-Arg-Gly-Asp-Ser-Pro-Lys-OH,
Bit-Gly-Gly-Gly-Lys-Thr-Ala-Asp-Cys(Trt)-Pro-OH,
cyclo-(Arg-Gly-Asp-D-Phe-Lys(N^{ε}-Bit))
cyclo-(Arg-Gly-Asp-D-Phe-Lys(N^{ε}-Aha)),
cyclo-(Arg-Gly-Asp-D-Phe-Lys(N^{ε}-Bit-Aha)-Gly),
cyclo-(Arg-Gly-Asp-D-Phe-Val-Lys(N^{ε}-Bit-Aha)),
cyclo-(Arg-Gly-Asp-D-Phe-N-Me-Lys(N^{ε}-Bit-Aha)),
cyclo-(Arg-Gly-Asp-D-Phe-Lys(N^{ε}-Bit-Aha)),
cyclo-(Arg-Gly-Asp-D-Phe-Lys(BOC-Aha)),
and the salts thereof.

2. Pharmaceutical preparation **characterized by** a content of at least one compound according to Claim 1 and/or one of its physiologically acceptable salts.

3. Compounds according to Claim 1 and their physiologically acceptable salts as integrin inhibitors to control pathologically angiogenic disorders, thromboses, myocardial infarction, coronary heart diseases, arteriosclerosis, tumours, osteoporosis, inflammations and infections.

4. Use of compounds according to Claim 1 and/or their physiologically acceptable salts for the production of a pharmaceutical.

5. Use of compounds according to Claim 1 for. purifying integrins by affinity chromatography.

6. Use of compounds according to Claim 1 as diagnostic markers for anti-biotin antibody reactions in ELISA-type assays and FACS analysis.

7. Use of compounds according to Claim 1 in atomic force microscopy to measure the strength of ligand-receptor interactions.

## Revendications

1. Composés choisis dans le groupe de ceux qui suivent:
Bit-Gly-Gly-Gly-Arg-Gly-Asp-Ser-Pro-Lys-OH,
Bit-Gly-Gly-Gly-Lys-Thr-Ala-Asp-Cys(Trt)-Pro-OH,
Cyclo-(Arg-Gly-Asp-D-Phe-Lys(N^{ε}-Bit)),
Cyclo-(Arg-Gly-Asp-D-Phe-Lys(N^{ε}-Aha)),
Cyclo-(Arg-Gly-Asp-D-Phe-Lys(N^{ε}-Bit-Aha)-Gly),
Cyclo-(Arg-Gly-Asp-D-Phe-Val-Lys(N^{ε}-Bit-Aha)),
Cyclo-(Arg-Gly-Asp-D-Phe-N-Me-Lys(N^{ε}-Bit-Aha)),
Cyclo-(Arg-Gly-Asp-D-Phe-Lys(N^{ε}-Bit-Aha)),
Cyclo-(Arg-Gly-Asp-D-Phe-Lys(BOC-Aha)),
ainsi que de leurs sels.

2. Préparation pharmaceutique **caractérisée en ce qu'**elle contient au moins un composé selon la revendication 1 et/ou un de ses sels physiologiquement acceptables.

3. Composés selon la revendication 1 et leurs sels physiologiquement acceptables en tant qu'inhibiteurs d'intégrine pour combattre les maladies pathologiquement angiogéniques, les thromboses, l'infarctus du myocarde, les maladies cardiaques coronariennes, l'artériosclérose, les tumeurs, l'ostéoporose, les inflammations et les infections.

4. Utilisation de composés selon la revendication 1 et/ou de leurs sels physiologiquement acceptables pour la fabrication d'un médicament.

5. Utilisation de composés selon la revendication 1 pour la purification des intégrines par chromatographie d'affinité.

6. Utilisation de composés selon la revendication 1 comme marqueurs de diagnostic pour les réactions anti-biotine-anticorps dans des tests de type ELISA et dans l'analyse FACS.

7. Utilisation de composés selon la revendication 1 dans la microscopie à champ de forces pour la mesure de la force des interactions ligand-récepteur.
